# EUROPEAN PATENT SPECIFICATION

(11) **EP 0 983 514 B1**
(45) Date of publication and mention of the grant of the patent: **19.12.2007**
(21) Application number: 98912691.7
(22) Date of filing: 02.04.1998
(51) Int. Cl.: G01N 33/94

(54) **A PROCESS AND A TEST KIT FOR HEROIN DETECTION**
VERFAHREN UND TESTKIT ZUM NACHWEIS VON HEROIN
PROCEDE ET KIT D'ESSAI POUR LA DETECTION DE L'HEROINE

(30) Priority: 10.04.1997 IL 12064397
(43) Date of publication of application: 08.03.2000
(73) Proprietor: Identa Ltd., 97280 Jerusalem (IL)
(72) Inventor: GLATTSTEIN, Baruch, 97280 Jerusalem (IL)
(74) Representative: TBK-Patent
(86) International application number: PCT/IL1998/000162
(87) International publication number: WO 1998/045714

(56) References cited:
- US-A- 4 806 487
- PATENT ABSTRACTS OF JAPAN vol. 095, no. 009, 31 October 1995 & JP 07 140130 A (KEISATSUCHIYOU CHOKAN), 2 June 1995,
- T SAKAI, N OHNO: "Spectrophotometric Determination of Stimulant Drugs in Urine by Color Reaction with Tetrabromophenolphthalein Ethyl Ester" ANALYTICAL SCIENCES, vol. 2, no. 3, June 1986, pages 275-279, XP002071888
- M J CHOI, E Y SONG, S KIM, J CHOI, D-S LHO, J PARK: "A Simple Device of the Dry Tetrabromophenolphthalein Ethyl Ester Reagent Strip for the Detection of Methamphetamine" ARCHIVES OF PHARMACAL RESEARCH, vol. 16, no. 3, September 1993, pages 227-230, XP002071889
- H A DINGJAN, S M DREYER-VAN DER GLAS, G T TJAN: "Colour tests for the identification of alkaloids (and related compounds)" PHARMACEUTISCH WEEKBLAD, vol. 115, no. 14, 4 April 1980, pages 445-467, XP002071890
- S P AGARWAL, N CHANDRASEKHARA, A U MADU: "A Colorimetric Method for the Determination of Amphetamines " ACTA PHARMACEUTICA TECHNOLOGICA, vol. 27, no. 3, 1981, pages 181-184, XP002071891

## Description

### FIELD OF THE INVENTION

The present invention relates to a process and test kit for the identification of heroin street drug. More specifically, the present invention relates to a process and test kit identification of heroin street drug without false positive with other street drugs. "Street drug" in this invention is defined as a drug in its free base form or as its mixture with its salt.

### BACKGROUND OF THE INVENTION

On numerous occasions a police officer has to determine whether or not a suspected material contains heroin and thus quickly establish probable cause. Often, the laboratory is closed, or many miles away, and the police officer has no way of making this determination. A test kit can help an officer to detect the presence of heroin or alternatively to determine that a tested sample definitely is not of said drug.

The quickest test known today for drug detection is a color test in which the response of the drug to a specific reagent makes it possible to assign the drug to one or more classes.

In order to obtain sufficient evidence to detain a suspected drug peddler or drug user, chemical spot test kits have been commercially developed and are used by many law enforcement agencies for the detection of narcotics and drugs of abuse.

Most of the commercial test kits for the identification of heroin are based on the famous Marquis sulfuric acid - formaldehyde reagent which gives a purple / violet characteristic color with morphinan structures, but not only with morphinan structures.

Clark, "Isolation and Identification of Drug" Pharmaceutical Press (1969) lists some 46 drugs that all respond in the same way to Marquis reagent. Furthermore, Marquis reagent gives other color with hundreds of drugs. It is thus apparent that this single color producing reagent cannot really serve for the field identification of heroin, because of the possibility of giving a false positive with other substances of similar structure or similar response characteristics.

The weakness of this single specific reagent test has led to the development of various combination tests which are intended to be used together in a manner which seeks to screen out the false positives and make possible a firm identification of heroin.

In U.S. Patent No. 3,955,926 there is described a process for the detection of narcotics wherein heroin is detected with a multicomponent reagent comprising a solution of benzylamine and iodic acid in dilute acid, and an absorbent support impregnated with ferric chloride, an alkali tartrate and alkali carbonate. This test, however is not specific enough for presumptive identification of heroin.

U.S. Patent No. 4,806,487 discloses the use of tetrabromophenolphetamine ethyl ester (TBPE) as a suitable color indicator of basic drugs. The use TBPE dissolved in 1,2-dichloroethane to detect the presence of methaphetamine, ephedrine and methylephedrine in urine samples is disclosed by Saki T. and Ohio N in Analytical Sciences vol 2 pp. 275-279 (1986).

In U.S. Patent No. 4,840,912 there is described a process for the detection of heroin street drug by a reagent that gives a color reaction to organic free base. It is based on the fact that heroin is not a pure salt, but has at least trace amounts of free base. This is because clandestine laboratories manufacture illicit drugs with no quality control.

In contradistinction thereto, medicinal drugs and pharmaceuticals containing an organic base are manufactured and sold as their pure salts. Manufacture is carried out in the pharmaceutical industry under strict quality control, so that these salts do not contain even trace amounts of free organic bases.

It was found that most of the heroin street drug contain trace amounts of free alkaloids bases. Now drug addicts turns to use heroin as a free base and not as a salt.

This said known process is carried out with a sulfonated aromatic pH indicator which produces a color reaction in the presence of trace amounts of organic free base. The sulfonated aromatic pH indicator is selected from the group consisting of bromophenol blue, bromochlorophenol blue and bromocresol green.

The test can be applied in two ways:
A. The material suspected as heroin street drug is placed on filter paper. A few drops of the indicator dissolved in organic solvent are added.
B. The filter paper is impregnated with the indicator by dipping it into a solution containing the indicator. The material suspected as heroin street drug is placed on the filter paper. Adding a few drops of organic solvent, for example dichloromethane, results in the staining of the paper in violet color with yellow background.

Trace amounts of water or exposing the filter paper to humidity causes the filter paper to be stained as heroin street drug. Therefore there was a need to make the filter paper hydrophobic. It was done by silanizing the filter paper. Silanizing reagents blocks the hydroxy groups of the cellulose. The filter paper becomes highly hydrophobic (water repellent). The preferred silanizing reagents are, for example, dimethylchlorosilane or trimethylchlorosilane. Mentioned indicators placed on the filter paper are not sensitive to moisture at all. This is the form of the commercial kit.

However, highly pure heroin, with no free base or trace amount of free base, will not react with this kind of indicators. Thus, in a case wherein the first test comes out negative it is proposed, in this U.S. patent, to apply a second reagent to turn the suspected substance to its free base form with volatile amine that will turn the suspected heroin salt to its base form and after the evaporation of the volatile amine, the indicator absorbed on the paper turns back to its yellow color.

By applying, in this method, the indicator once again, a violet color would be presumpive test for detection of alkaloids.

As an alternative to the sulfonated pH indicator, there is provided a second color reagent that reacts with organic free base in anhydrous conditions.

The known commercial kit contains only the pH sulphonated indicator absorbed on hydrophobic filter paper and the tedious procedure for detecting salts containing organic base was abandoned.

The above mentioned U.S. patent has several disadvantages:
1. The heroin drug testing kit is based on the fact that street drug heroin has at least trace amounts of heroin in its free base form. However, heroin is not the only street drug manufactured in clandestine laboratories that contains organic base. The other illicit wide spread drugs, containing organic base are: PCP (Phencyclidine), Amphetamine, Methamphetamine,3,4-Methylenedioxyamphetamine (MDA), 3,4-Methylenedioxymethamphetamine (MDMA, known as "Extasy"),3,4-Methylenedioxyethylamphetamine (MDEA, known as "Eve"), and 3,4-Methylenedioxy-N-ethylamphetamine (MDE). All these drugs can react as its organic free base. The commercial kits use this indicator as a general indicator to the presence of organic base in illicit drugs.
2. It was found that the commercial kit (where the indicator is placed on hydrophobic filter paper) reacts surprisingly with some drugs even where the drugs are in their pure salt form such as: Methamphetamine hydrochloride and diphenhydramine hydrochloride.
3. The sulfonated pH indicators cannot detect organic base in a pure salt.
4. The sulfonated indicators react to the above mentioned street drugs in the same purple color.
5. Trace amounts of water react with the sulfonated indicators as heroin.

Surprisingly, the disadvantages of this sulfonated pH indicator has led the inventor to the development of another specific reagent for the heroin street drug. This reagent is intended to be used as a general indicator for drugs containing organic free base manufactured in clandestine laboratories. It then also gives a distinct color to a second reagent, that screens out the amphetamine, MDA and other street drugs manufactured in clandestine laboratories, that do not give the same color reaction as heroin.

Saki, T. and Ohio, N. (1986), Analytical Sciences 2:275-279 discloses the use of an indicator solution comprising tetrabromophenolphthalein ethyl ester (TBPE) dissolved in 1,2-dichloroethane to detect methamphetamine, ephedrine and methylephedrine in urine samples.

US-A-4,806,487 discloses TBPE as a suitable colour indicator of basic drugs as heroin, phencyclidine (PCP) or amphetamine.

### SUMMARY OF THE INVENTION

The present invention there provides a process for the detection of heroin street drug and a differentiation of heroin street drug from other substances that may react to a free base indicator.

This is accomplished by applying to a sample of the suspected drug a reagent which gives a characteristic color reaction in the presence of trace amounts of free base and applying a second color reaction that differentiates between heroin street drug and other illicit drugs manufactured in clandestine laboratories.

The present invention also provides a test kit for heroin detection comprising of;
a) a lipophilic pH indicator solution wherein the indicator is dissolved in a solvent immiscible with water and said indicator is characterized by having a distinctive color response to heroin, amphetamine and MDA street drugs containing organic base and by having another color response to the other street drugs manufactured in clandestine laboratories.
b) a Marquis reagent wherein by using this reagent the heroin gives a purple color and amphetamine methamphetamine and MDA gives an orange color. PCP does not give a color reaction with the reagent.

### DETAILED DESCRIPTION OF THE INVENTION

Contrary to the prior art approach for the detection of the heroin street drug, it has now been surprisingly found that a powder suspected of being heroin can be detected and can be differentiated from other illicit drugs, containing organic base and manufactured in clandestine laboratories.

As mentioned before, existence of organic free base in a suspected substance is an indication for an illicit drug manufactured in a clandestine laboratory. An organic free base can be detected by an appropriate indicator. Sulfonephthalein indicators are a good option, because they react well to a free organic base when the indicator is dissolved in an organic solvent. These include the following sulfonephthaleins: bromophenol blue, bromocresol green and bromocresol purple. However, the water solubilizing sulfonic acid group that is present in the sulfonephthalein cause the indicator to react with trace amounts of water and to give the same response (color) with water and with heroin. Therefore, the sulfonephthaleins are unsuited for (use in this field) for these types of testing kits.

The preferred indicator used in the present invention is a lipophilic pH indicator such as Tetrabromophenolphthalein ethyl ester that has a very good solubility in organic solvents, gives sensitive color changes and is completely insoluble in water. It intentionally lacks the water solubilizing sulfonic acid group that is present in the sulphonephthaleins. Tetrabromophenolphthalein ethyl ester is the analog to bromophenol blue.

The color reaction of the lipophilic indicator is based on two stages;
A) A small amount of the indicator is dissolved in a solvent such as chloroform, dichloromethane, or 1,2-dichloroethane that is immiscible with water.
   A trace amount of the suspected heroin is placed in a test tube and a few drops of the indicator are added and the solution is observed for any color change. If an organic free base is present, there will be a change in the color of the solution (depending on the characteristics of the indicator and the basicity of the base).
   In this stage a positive reaction will be an indication for the suspected drug containing an organic base, and manufactured in clandestine laboratories. These suspected illicit drugs are: Heroin, PCP, Amphetamine, Methamphetamine, 3,4-Methylenedioxyamphetamine (MDA), 3,4-Methylenedioxymethamphetamine (MDMA), 3,4-Methylenedioxyethylamphetamine (MDEA), and 3,4-Methylenedioxy-N-ethylamphetamine (MDE).
   Heroin, amphetamine and MDA will give an orange to red color with the indicator solution and the other drugs will give a violet color reaction. Adding hexane will cause the orange color to turn back to yellow and the red color reverts back to an orange or yellow. On the other hand, all the other suspected drugs will give a violet color, and by the addition of hexane, there will not be any change in the solution color.
B) By using Marquis reagent, only heroin will give a characteristic violet/purple color. Amphetamine methamphetamine and MDA will give an orange color. PCP does not give a color reaction
C) If a change of color is not seen in stage A, a few drops of buffer 7 are added (if there is any salt of an organic base, the buffer 7 partitions the organic base of the salt to the organic layer), if there is no color change, the suspected substance does not contain a salt of organic base and there is no need to use the Marquis test.

### EXAMPLE 1:

A. About 30 mg of Tetrabromophenolphthalein ethyl ester (TBP) is dissolved in 100 ml of dichloromethane chloroform (or 1,2, dichloroethane). The solution is yellow. A trace amount of suspected heroin street drug is added to a small test tube. A 0.5 ml of TBP reagent is added to the test tube and an immediate orange to red color will develop. This will be an indicator to heroin, Amphetamine and MDA street drug. The orange or red color depends on the concentration of the heroin in the suspected material. In a case of low concentration, an orange color will appear. With high concentrations, a red color will appear.
B. Addition of 1 ml hexane causes the color to change from red to orange or yellow and from orange to yellow (other street drugs give a violet color that does not change after adding hexane).
C. Marquis test will give a purple color only to heroin.
D. If there is no color change in stage A, a few drops of aqueous buffer 7 solution are added. If there is a color change, the suspected substance contains a salt of organic base. If there is no color change, the substance does not contain a salt of organic base at all.

## Claims

1. A process for the detection of heroin street drug and differentiation of heroin street drug from other substances that may react to a free base indicator which comprises the steps of;
a) applying the lipophilic indicator tetrabromophenolphtalein ethyl ester (TBPE) dissolved in organic solvent immiscible with water, to a sample of the suspected drug providing an orange to red color with heroin, amphetamine and 3,4-methylenedioxyamphetamine (MDA) and violet/purple color with the other drugs; and
b) applying to the suspected sample Marquis sulfuric acid formaldehyde reagent that differentiates between heroin street drug and the other drugs, providing a purple/violet color with heroin and an orange color with amphetamine, methamphetamine and MDA.

2. A process according to claim 1 wherein the organic solvent, in which TBPE is dissolved, is selected from chloroform, dichloromethane or 1,2-dichloroethane.

3. A process according to claim 1 wherein adding hexane to the suspected sample after the first color reaction causes the color to change from red, to orange or yellow, and from orange, to yellow, if there is heroin, amphetamine and MDA in the sample, and if there is one of the other drugs there will be no change in the violet color.

4. A test kit for heroin street drug detection and differentiation of heroin street drug from other substances that may react to a free base indicator according to the process of any of the preceding claims comprising of;
a) Tetrabromophenolphtalein ethyl ester (TBPE) dissolved in a solvent immiscible with water **characterized by** having a distinctive color response to heroin, amphetamine and MDA street drugs containing organic base and by giving another color to the other street drugs manufactured in clandestine laboratories;
b) a Marquis reagent wherein by using this reagent the heroin gives a purple/violet color and amphetamine, methamphetamine and MDA gives an orange color;
c) hexane.

5. A test kit according to claim 4 wherein the TBPE is dissolved in an organic solvent selected from chloroform, dichloromethane or 1,2-dichloroethane.

## Patentansprüche

1. Verfahren zum Nachweis von Straßendroge Heroin und Unterscheidung von Straßendroge Heroin von anderen Substanzen, welche mit einem Freie-Base-Indikator reagieren können, welches die Schritte enthält;
a) Anwenden des lipophilen, in organischem, mit Wasser nicht mischbarem Lösungsmittel gelösten Indikators Tetrabromophenolphtaleinethylester (TBPE) auf eine Probe der verdächtigen Droge,das eine orange bis rote Farbe mit Heroin, Amphetamin und 3,4-Methylendioxyamphetamin (MDA) und eine lila/violette Farbe mit anderen Drogen bildet; und
b) Anwenden von Marquis-Schwefelsäure-Formaldehyd-Reagenz, das zwischen Straßendroge Heroin und anderen Drogen unterscheidet, auf die verdächtige Probe, das eine lila/violette Farbe mit Heroin und einer orange Farbe mit Amphetamin, Methamphetamin und MDA bildet.

2. Verfahren nach Anspruch 1, wobei das organische Lösungsmittel, in welchem TBPE gelöst ist, aus Chloroform, Dichlormethan oder 1,2-Dichlorethan ausgewählt ist.

3. Verfahren nach Anspruch 1, wobei das Hinzufügen von Hexan zu der verdächtigen Probe nach der ersten Farbreaktion dazu führt, dass sich die Farbe von Rot nach Orange oder Gelb und von Orange zu Gelb ändert, wenn es dort in der Probe Heroin, Amphetamin und MDA gibt, und wenn es dort eine der anderen Drogen gibt, wird es keine Änderung in der violetten Farbe geben.

4. Testkit für Straßendroge Heroin-Nachweis und Unterscheidung der Straßendroge Heroin von anderen Substanzen, die mit einem Freie-Base-Indikator gemäß dem Verfahren nach einem der vorangehenden Ansprüche reagieren kann, enthaltend,
a) in organischem, mit Wasser nicht mischbarem Lösungsmittel gelöstes Tetrabromophenolphtaleinethylester (TBPE), **gekennzeichnet durch** Aufweisen einer charakteristischen Farbantwort auf eine organische Base enthaltende Heroin, Amphetamin und MDA Straßendrogen und **durch** Ergeben einer anderen Farbe auf die sonstigen, in verborgenen Laboratorien gefertigten Straßendrogen;
b) ein Marquis-Reagenz, wobei **durch** das Verwenden dieses Reagenzes das Heroin eine lila/violette Farbe ergibt und Amphetamin, Methamphetamin und MDA eine orange Farbe ergeben;
c) Hexan

5. Testkit nach Anspruch 4, wobei das TBPE in einem aus Chloroform, Dichlormethan oder 1,2-Dichlorethan ausgewählten, organischen Lösungsmittel gelöst ist.

## Revendications

1. Procédé de détection de drogue héroïne illicite et différenciation de la drogue héroïne illicite vis-à-vis d'autres substances qui peuvent réagir à un indicateur de base libre qui comprend les étapes de :
a) application de l'indicateur lipophile ester éthylique de tétrabromophénolphtaléine (TBPE) dissous dans un solvant organique non miscible dans l'eau, à un échantillon de la drogue suspectée fournissant une couleur orange à rouge avec l'héroïne, l'amphétamine et la 3,4-méthylènedioxyamphétamine (MDA) et une couleur violet/pourpre avec les autres drogues ; et
b) l'application à l'échantillon suspecté de réactif formaldéhyde acide sulfurique de Marquis qui effectue une différenciation entre la drogue héroïne illicite et les autres drogues, en fournissant une couleur pourpre/violet avec l'héroïne et une couleur orange avec l'amphétamine, la méthamphétamine et la MDA.

2. Procédé selon la revendication 1, dans lequel le solvant organique, dans lequel du TBPE est dissous, est choisi parmi le chloroforme, le dichlorométhane ou le 1,2-dichloroéthane.

3. Procédé selon la revendication 1, dans lequel l'ajout de l'hexane à l'échantillon suspecté après la première réaction de couleur provoque le changement de couleur du rouge à l'orange ou au jaune, et de l'orange, au jaune, s'il existe de l'héroïne, de l'amphétamine et de la MDA dans l'échantillon, et s'il existe l'une des autres drogues il n'y aura pas de changement en la couleur violette.

4. Kit de test pour la détection de la drogue héroïne illicite et la différenciation de la drogue héroïne illicite vis-à-vis d'autres substances qui peuvent réagir à un indicateur de base libre selon le procédé selon l'une quelconque des revendications précédentes composté :
a) d'ester éthylique de tétrabromophénolphtaléine (TBPE) dans un solvant non miscible dans l'eau **caractérisé par le fait qu'**il a une réponse colorée particulière aux drogues illicites héroïne, amphétamine et MDA contenant une base organique et qu'il donne une autre couleur face aux autres drogues illicites fabriquées dans des laboratoires clandestins ;
b) d'un réactif de Marquis dans lequel par l'utilisation de ce réactif l'héroïne donne une couleur pourpre/violet et l'amphétamine, la méthamphétamine et la MDA donnent une couleur orange ;
c) de l'hexane.

5. Kit de test selon la revendication 4, dans lequel le TBPE est dissous dans un solvant organique choisi parmi le chloroforme, le dichlorométhane ou le 1,2-dichloroéthane.
